# FASCICULE DE BREVET EUROPEEN

(11) **EP 0 609 132 B1**
(45) Date de publication et mention de la délivrance du brevet: **16.04.1997**
(21) Numéro de dépôt: 94400149.4
(22) Date de dépôt: 25.01.1994
(51) Int. Cl.: A61K 7/48, A61K 7/027, A61K 7/021

(54) **Dispersion solide anhydre contenant des composés organofluorés hydrocarbonés et son utilisation en cosmétique**
Wasserfreie feste Dispersion, die Organofluorkohlenwasserstoffverbindungen enthält und ihre Verwendung in der Kosmetik
Anhydrous solid dispersion containing organofluorinated hydrocarbons and its use in cosmetic

(30) Priorité: 25.01.1993 FR 9300911
(43) Date de publication de la demande: 03.08.1994
(73) Titulaire: L'OREAL, 75008 Paris (FR)
(72) Inventeur: Miguel, Dolorès, F-92340 Bourg-la-Reine (FR)
(74) Mandataire: Casalonga, Axel

(56) Documents cités:
- EP-A- 0 196 904
- EP-A- 0 390 206
- EP-A- 0 494 412
- EP-A- 0 524 892
- EP-A- 0 545 786
- WO-A-93/11103
- US-A- 5 108 737
- PATENT ABSTRACTS OF JAPAN vol. 15, no. 068 (C-0807)18 Février 1991 & JP-A-02 295 913 (SHISEIDO CO LTD) 6 Décembre 1990
- PATENT ABSTRACTS OF JAPAN vol. 016, no. 040 (C-0906)31 Janvier 1992 & JP-A-03 246 211 (KAO CORP.) 1 Novembre 1991
- PATENT ABSTRACTS OF JAPAN vol. 016, no. 315 (C-0961)10 Juillet 1992 & JP-A-04 089 422 (KAO CORP.) 23 Mars 1992
- PATENT ABSTRACTS OF JAPAN vol. 012, no. 198 (C-502)8 Juin 1988 & JP-A-63 002 916 (KANEBO LTD) 7 Janvier 1988

## Description

La présente invention concerne des dispersions solides anhydres de polyols dans un milieu anhydre contenant des composés organofluorés hydrocarbonés ainsi que leur utilisation en cosmétique.

Dans le domaine de la cosmétique, des produits cosmétiques gras solides sont usuellement utilisés pour fabriquer des rouges à lèvres et sont alors obtenus en durcissant des corps gras et, pour fabriquer des produits comme les fonds de teint que l'on applique à l'éponge, ils sont alors obtenus en mélangeant et compactant des poudres et des agents gras.

Dans la demande de brevet japonais n° (de publication) 1-143.812, il est décrit l'incorporation d'une quantité importante d'alcools polyvalents dans une émulsion solide à usage cosmétique par le biais d'une huile de silicone et d'un organopolysiloxane de polyoxyalkylène modifié en tant qu'agent émulsifiant. Avec ce système, il est cependant difficile d'obtenir une bonne homogénéité de la formule.

Ces produits présentent l'inconvénient de ne pas être hydratants.

Pour préparer des compositions hydratantes, il est apparu indispensable de savoir disperser des alcools polyhydriques dans un milieu anhydre, mais ces produits des tendance à se couvrir de gouttelettes de corps gras, notamment en atmosphère humide et ce phénomène d'exsudation est un désavantage car il provoque une forte répulsion de la part des utilisatrices.

La présente invention pallie à l'inconvénient de l'exsudation des dispersions solides d'alcools polyhydriques dans un milieu cosmétique gras. De manière surprenante, on a ainsi constaté que l'introduction de composés organofluorés du type fluorohydrocarboné permet de résoudre le problème d'exsudation des dispersions solides contenant des alcools polyhydriques dans une phase grasse.

L'invention est définie par les revendications.

La présente invention concerne des dispersions solides anhydres comportant de 20 à 95% d'un corps constitué de 10 à 50% en poids d'au moins une cire de point de fusion supérieur à 55°C et de 0,5 à 50% en poids d'un alcool polyhydrique dispersé ainsi que 0,1 à 50% en poids de composés organofluorés hydrocarbonés.

Par dispersion solide, on entend une composition solide entre 0 et 50°C, ce qui correspond au domaine de température de stockage et d'utilisation usuelle des produits cosmétiques.

Les organofluorés sont du type fluorohydrocarbure, non volatils, de point d'ébullition supérieur à 30°C.

Le terme fluorohydrocarbure désigne des composés dont la structure chimique comporte un squelette carboné dont certains atomes d'hydrogène ont été substitués par des atomes de fluor. Le squelette carboné peut comporter un ou plusieurs hétéroatomes et/ou un ou plusieurs groupements organiques fonctionnels.

Pour les fluorohydrocarbures, on définit le taux de substitution des atomes d'hydrogène par des atomes de fluor, sous la forme du rapport : nombre d'atomes de fluor/(nombre d'atomes de fluor + nombre d'atomes d'hydrogène) où seuls les atomes d'hydrogène liés aux atomes de carbone du squelette sont pris en compte. Les fluorohydrocarbures ou huiles fluorohydrocarbonées de l'invention comportent au moins un groupement hydrocarboné dans la molécule.

Les fluorohydrocarbures de l'invention ont pour formule la formule I suivante :

(R_{F})ₓ - (A)_{y} - (R_{H})_{z} (I)

dans laquelle :
x représente 1, 2 ou 3,
y représente 0 ou 1,
z représente 0, 1, 2 ou 3,
à la condition que y et z ne soient pas simultanément 0, et que lorsque z est 0, x est 2 ou 3,
R_{F} représente un radical fluoré aliphatique ou aromatique, saturé ou insaturé, à chaîne linéaire, ramifiée ou cyclique, cette chaîne pouvant être fonctionnalisée et/ou être interrompue par des atomes divalents tels que l'oxygène ou le soufre, ou trivalents tels que l'azote et/ou substituée par des atomes d'hydrogène ou d'autres atomes d'halogène, à la condition que, pour deux atomes de carbone du squelette, ne soit pas présent plus d'un de ces substituants autres que le fluor,
R_{H} représente un radical hydrocarboné aliphatique ou aromatique, saturé ou insaturé, à chaîne linéaire, ramifiée ou cyclique, cette chaîne pouvant être fonctionnalisée et/ou interrompue par un ou plusieurs atomes divalents tels que l'oxygène ou le soufre ou par un ou plusieurs atomes trivalents comme l'azote,
A représente un radical di, tri ou quadrivalent tel que 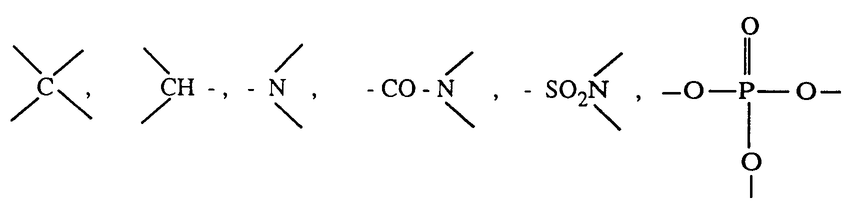 les structures cycliques, aliphatiques ou aromatiques ou les insaturations éthyléniques.

Par fonctionnalisé, on entend selon l'invention, une substitution du squelette, intercalaire, terminale ou pendante, par au moins un groupement organique fonctionnel comme une fonction alcool, thiol, acide, carbonyle, sulfoxyde, ester, amide, amine, phosphate, éthylénique, acéthylénique, et énamine ou sulfonamide.

Par insaturation éthylénique, on entend par exemple

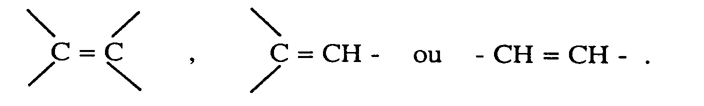

De préférence, R_{H} représente un radical alkyle linéaire ou ramifié en C₁-C₂₂ ou un mélange de radicaux alkyle linéaires ou ramifiés en C₁-C₂₂, un radical aryle en C₆-C₁₀ ou un radical aralkyle en C₇-C₁₅.

De préférence, R_{F} représente un radical perfluoroalkyle ayant de 4 à 22 atomes de carbone.

Selon l'invention, les fluorohydrocarbures utilisés ont de préférence un taux de substitution compris entre 10 et 90 %. De façon préférée, ce taux est supérieur à 20 % et inférieur à 80 %.

A titre illustratif, on peut citer les composés possédant des groupes perfluorocarbures et des groupes hydrocarbures, le nombre total de carbone étant compris entre 10 et 30, le nombre d'atomes de carbone des groupes hydrocarbures étant égal ou supérieur à deux fois le nombre d'atomes de carbone des groupes perfluorocarbures, tels qu'ils sont décrits dans le document JP 63-002916.

De même, à titre illustratif, on peut citer les fluorohydrocarbures décrits dans la demande de brevet FR 91-15019 et dont la structure générale est définie par la formule (III):

R₁ - (CH₂)ₙ - X - [C₃H₅(OH)] - (Y)ₓ - R₂ (III)

dans laquelle C₃H₅(OH) représente les structures : 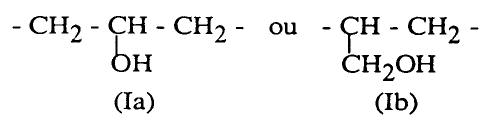
R₁ représente un radical alkyle perfluoré en C₄-C₂₀ ou un mélange de radicaux alkyle perfluorés en C₄-C₂₀;
R₂ représente un radical alkyle linéaire ou ramifié en C₁-C₂₂ ou un mélange de radicaux alkyle linéaires ou ramifiés en C₁-C₂₂ ou un radical aryle ou aralkyle;
n est compris entre 0 et 4;
X represente O, S, 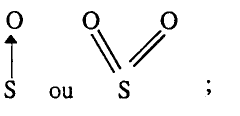
x représente O ou 1 ;
Y représente O, S, 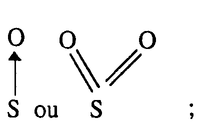
sous réserve que lorsque X = 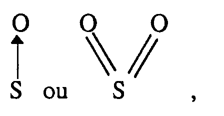
Y n'est pas

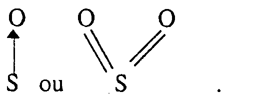

Les composés peuvent être préparés soit par la réaction d'un composé fluoré à hydrogène acide de formule (II) :

R₁ - (CH₂)ₙ - X - H (II)

avec un époxyde de formule (II') : 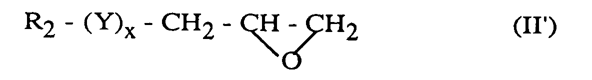 ou par la réaction d'un composé hydrocarboné à hydrogène acide de formule (IV) :

R₂ - (Y)ₓ - H (IV)

avec un époxyde fluoré de formule (V) : 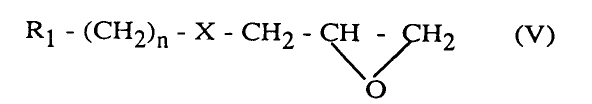 dans lesquelles R₁, R₂, n et x ont les significations ci-dessus,
X désigne O ou S, Y désigne O ou S,
en présence d'un composé basique ou acide jouant le rôle de réactif ou de catalyseur; l'oxydation éventuelle de la fonction mercaptan en sulfoxyde ou sulfone avec de l'eau oxygénée et la récupération du composé de formule (I) obtenu.

Ces composés sont décrits dans WO 93/11103 et EP 166.696.

Comme exemples de tels composés, on peut citer le 1-(2'-F-hexyléthylthio)-3-(2''-éthylhexyloxy)2-propanol, le 1-(2'-F-octyléthylthio)3-(2''-éthylhexyloxy)-2-propanol, le 1-(2'-F-octyléthylthio)3-butyloxy-2-propanol, le 1-(2'-F-octyléthylthio)3-phénoxy-2-propanol, le 1-(2'-F-hexyléthylthio)3-dodécyloxy-2-propanol, le 1-(2'-F-hexyléthylthio)2-décanol, le 1-(2'-F-hexyléthylthio)2-hexanol, le 1-(2'-F-octyléthylthio)-2-hexanol et le 1-(2'-F-hexyléthyloxy)-3-(2''-éthylhexyloxy)-2-propanol.

Par ailleurs, on peut également utiliser, selon l'invention, les composés de formule (IV') :

R_{F} - (CH₂)ₙ - X - [C₃H₅(OH)] - Y - (CH₂)ₘ - R'_{F} (IV')

dans laquelle C₃H₅ (OH) représente les structures : 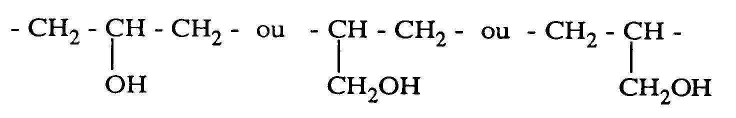
R_{F} et R'_{F}, identiques ou différents, représentent un radical alkyle perfluoré, linéaire ou ramifié en C₄-C₂₀ ou un mélange de radicaux alkyle perfluorés, linéaires ou ramifiés en C₄-C₂₀;
m et n, identiques ou différents, représentent 0, 1, 2, 3 ou 4;
X et Y, identiques, sont - O - ou - S -.

Ces composés sont décrits dans DE-2.702.607, JP 89-193.236, JP 92-275.268 et US-3.893.984.

On peut utiliser aussi les composés de formule :

R_{F} - (CH₂)ₙ - X - [C₃H₅(OH)] - Y - (CH₂)ₘ - R'_{F} (I')

dans laquelle C₃H₅(OH) représente les structures : 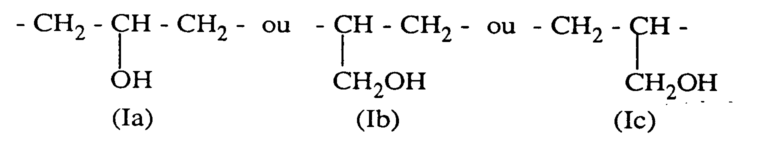
R_{F} et R'_{F}, identiques ou différents, représentent un radical alkyle perfluoré, linéaire ou ramifié en C₄-C₂₀ ou un mélange de radicaux alkyle peffluorés, linéaires ou ramifiés en C₄-C₂₀;
m et n, identiques ou différents, représentent 0, 1, 2, 3 ou 4 et X est O et Y est S ou X est S et Y est O.

Les composés de formule (I') peuvent être préparés en mettant en oeuvre la réaction d'un composé fluoré à hydrogène acide de formule :

R_{F} - (CH₂)ₙ - X - H

avec un époxyde de formule : 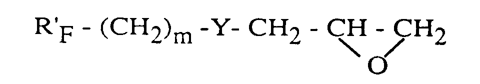 ou la réaction d'un composé fluoré à hydrogène acide de formule :

R_{F'} - (CH₂)ₘ - Y - H

avec un époxyde fluoré de formule : 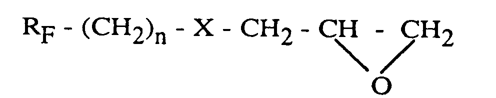 en présence d'un composé basique ou acide jouant le rôle de réactif ou de catalyseur. Les composés sont décrits dans FR 9306605.

On peut aussi utiliser, selon l'invention, les composés décrits dans le document DE 2.052.579, de formule : 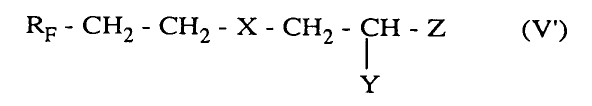
où Y est OH, et
Z est

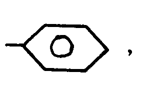

   - CH_{3'} - CH₂OH, - CH₂OCOCH₃
ou bien Y est - CH₂OH et Z est -O-COCH₃
X représente - O - , - S - , 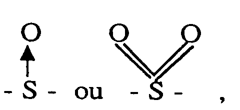 et
R_{F} représente un radical alkyle perfluoré, linéaire ou ramifié, en C₄-C₂₀, ou un mélange de radicaux alkyle perfluorés, linéaires ou ramifiés, en C₄-C₂₀;
ou bien les composés décrits dans le document US 3.952.066, de formule :

R_{F} - CH = CH - CH₂ - O - CH₂ - [C₂H₄ - OW] (VI')

où
C₂H₄OW désigne : 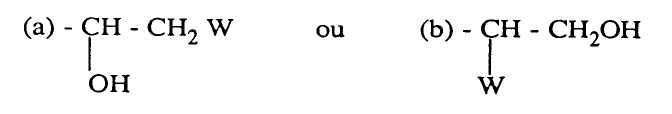
W désignant : -OR, -SR, -COOR, 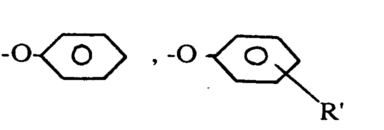
R désigne un radical alkyle en C₁-C₁₈, linéaire ou ramifié,
R' désigne -CH₃ ou -OH, en position ortho ou para, et
R_{F} représente un radical alkyle perfluoré, linéaire ou ramifié, en C₄-C₂₀, ou un mélange de radicaux alkyle perfluorés, linéaires ou ramifiés, en C₄-C₂₀.

Par ailleurs, on peut citer, à titre d'exemple, les produits vendus sous la dénomination de NOFABLE FO par la Société NIPPON OIL & Co, ayant la formule suivante : 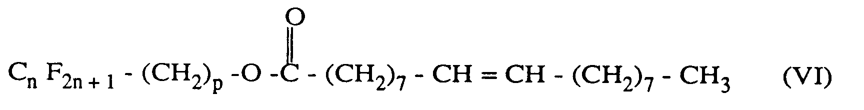 dans laquelle n est un nombre entier égal à 6 ou 8 et p est 1 ou 2.

Les composés préférés sont ceux de formule (VI) et ceux de formule (III), et plus particulièrement ceux de formule (IV) pour lesquels X est S, x est 1 et Y est O et X est S et x est O.

Selon une forme de réalisation préférée, la dispersion solide selon l'invention peut également contenir jusqu'à 10% d'une charge minérale ou organique telle que le talc, l'amidon, par exemple.

Il est également possible que la dispersion selon l'invention contienne jusqu'à environ 30% (de préférence de 0,1 à 20%) d'au moins un pigment et de 0 à 20% (de préférence de 0,5 à 10%) d'au moins un agent tensio-actif.

Parmi les tensio-actifs habituellement utilisés dans les rouges à lèvres, on peut mentionner les tensio-actifs anioniques ou nonioniques, de HLB (hydrophilic-lipophilic Balance) inférieur à 10 (préférentiellement inférieur à 5), à l'exclusion des tensio-actifs siliconés. Parmi les tensio-actifs non-ioniques, on peut citer les lécithines, les succinylglycérides.

Parmi les tensio-actifs anioniques, on peut citer les alkylphosphates, le lanolate de magnésium, le lanolate de zinc, le lanolate de cuivre, le lanolate d'arginine et l'octadécanoate de magnésium.

L'emploi d'un tensio-actif permet, en effet, d'obtenir notamment une dispersion plus fine et plus stable.

Selon un mode de réalisation plus particulièrement préféré, il est possible d'ajouter à la dispersion selon l'invention une quantité suffisante d'un polymère pour la stabiliser davantage contre l'exsudation en atmosphère humide.

De tels polymères sont de préférence liposolubles et possèdent un taux de motifs hydrophiles très faible.

Parmi ceux-ci, on peut citer les polyalkylènes (notamment les polyéthylènes et polybutènes), les polyacrylates et les polymères siliconés compatibles avec les corps gras tels que le polystéanylméthylsiloxane vendu par la Société GOLDSCHMIDT sous la dénomination ABIL WAX 9800.

Parmi les polyalkylènes, on peut citer le polybutène, notamment celui vendu par la Société AMOCO sous la dénomination INDOPOL.

Selon l'invention, l'alcool polyhydrique peut être un composé ayant de 2 à 8 atomes de carbone et de 2 à 6 fonctions hydroxyle. Parmi ces composés, on peut citer l'éthylèneglycol, le glycérol, le propanediol-1,2, la diglycérine, l'érythritol, l'arabitol, l'adonitol, le sorbitol ou le dulcitol.

L'alcool polyhydrique peut également être un polyéther alcool de poids moléculaire moyen compris entre 150 et 600 et parmi ceux-ci, on peut citer le polyéthylèneglycol 300 et la polyglycérine 500.

Selon l'invention, lorsque le composé organofluoré hydrocarboné est fonctionnalisé avec des groupements OH, SH, NH ou NH₂, le polyol comporte au moins trois fonctions hydroxyle.

La phase d'alcool polyhydrique de la dispersion selon l'invention peut être enrichie par des actifs hydrosolubles tels que les acides aminés (par exemple l'Arginine, la Lysine, l'hydroxyproline, la Proline et la Sérine), les vitamines solubles dans la glycérine telle que le D, L-Panthénol et les filtres solaires compatibles. Ceux-ci peuvent être présents à raison de 0,05 à 5%.

Le corps gras selon l'invention est constitué de 10 à 50% en poids d'au moins une cire dont le point de fusion est supérieur à 55°C, le reste étant soit une cire de point de fusion inférieur à 55°C, soit une huile ou un mélange de ceux-ci, le point de fusion finissant de tout mélange doit être inférieur à 110°C, ce qui n'empêche pas que certains constituants du mélange puissent présenter un point de fusion supérieur.

Parmi les cires ayant un point de fusion supérieur à 55°C, susceptibles d'être utilisées selon l'invention, on peut mentionner les cires animales, végétales, minérales, synthétiques et les fractions diverses de cires naturelles, toutes ces cires ayant, en règle générale, un point de fusion compris entre 55 et 110°C, et une pénétration à l'aiguille à 25°C comprise entre 3 et 40, telle que mesurée selon la norme américaine ASTM D5 ou selon la norme française NFT 004.

Le principe de la mesure de la pénétration d'une aiguille selon ces deux normes consiste à mesurer la profondeur exprimée en dixièmes de millimètre, à laquelle pénètre une aiguille normalisée (pesant 2,5 g, placée dans un porte-aiguille pesant 47,5 g, soit au total 50 g), placée sur la cire pendant 5 secondes.

Parmi les cires animales que l'on peut utiliser, on peut citer entre autres les cires d'abeilles, les cires de lanoline et les dérivés issus de lanoline. Parmi les cires végétales, on peut citer, entre autres, les cires de Carnauba, de Candellila, d'Ourcurry, les cires de fibres de liège, les cires de canne à sucre et les cires du Japon. Parmi les cires minérales, on peut citer, en particulier, les paraffines, les cires microcristallines, les cires de lignite et les ozokérites. Parmi les cires synthétiques, on peut citer, en particulier, les cires de polyéthylène, les cires obtenues par synthèse de Fisher et Tropsch et les polymères cireux ainsi que leurs esters. Toutes ces cires sont bien connues de l'homme de l'art.

Parmi les huiles susceptibles d'être utilisées en mélange avec les cires, on peut en particulier citer :
- les huiles minérales telles que l'huile de paraffine, l'huile de vaseline et les huiles minérales ayant un point d'ébullition compris entre 310 et 410°C;
- les huiles d'origine animale telles que le perhydrosqualène;
- les huiles végétales telles que l'huile d'amande douce, l'huile de calophyllum, l'huile de palme, l'huile d'avocat, l'huile de jojoba, l'huile d'olive, l'huile de ricin, les huiles de germes de céréales telles que l'huile de germes de blé;
- les huiles de silicone telles que le diméthylpolysiloxane;
- les esters de synthèse tels que l'huile de Purcelin, le myristate de butyle, le myristate d'isopropyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate de décyle, le laurate d'hexyle, le dicaprylate de propylèneglycol, l'adipate de di-isopropyle;
- les alcools organiques compatibles avec les corps gras tels que l'alcool oléique, l'alcool linoléique, l'alcool linolénique, l'alcool isostéarylique, l'octyl dodécanol;
- les esters dérivés d'acide lanolique tels que le lanolate d'isopropyle, le lanolate d'isocétyle.

Parmi les huiles, on peut également citer les acétylglycérides, les octanoates et décanoates d'alcools et de polyalcools tels que ceux de glycol et de glycérol, les ricinoléates d'alcools et de polyalcools tels que celui de cétyle.

On peut ajouter comme corps gras les huiles hydrogénées concrètes à 25°C telles que l'huile de ricin hydrogénée, l'huile de palme hydrogénée, le suif hydrogéné, l'huile de coco hydrogénée, les esters gras concrets à 25°C tels que le myristate de propylèneglycol et le myristate de myristyle, l'alcool cétylique, les mono-, di- ou triglycérides et les sucroglycérides.

En outre, cette phase grasse peut contenir des pigments. Comme pigments colorés, on peut mentionner le noir de carbone ou l'oxyde de fer noir, les oxydes de chrome, les oxydes de fer jaune et rouge, les outremers (polysulfures d'aluminosilicates), le pyrophosphate de manganèse, le bleu ferrique, le dioxyde de titane et enfin certaines poudres métalliques telles que celles d'argent ou d'aluminium. Les pigments sont le plus souvent utilisés en mélange avec des agents nacrants tels que l'oxychlorure de bismuth, le mica-titane, les cristaux de guanine et certains colorants organiques tels que le carmin de cochenille et les laques organiques.

Ces laques qui sont couramment employées pour conférer aux lèvres et à la peau un effet de maquillage, sont des sels de calcium, de baryum, d'aluminium ou de zirconium, de colorants acides tels **q**ue les colorants halogéno-acides, azoïques, anthraquinoniques, par exemple.

Parmi ces laques, on peut en particulier citer celles connues sous les dénominations de D and C Red 21, D and C Orange 5, D and C Red 27, D and C Orange 10, D and C Red 3, D and C Red 7, D and C Red 2, D and C Red 4, D and C Red 8, D and C Red 33, D and C Yellow 5, D and C Yellow 6, D and C Green 5, D and C Yellow 10, D and C Green 3, D and C Blue 1, D and C Blue 2, D and C Violet 1, par exemple.

Les compositions cosmétiques de maquillage selon l'invention peuvent également contenir des agents antioxydants à raison de 0 à 3%, de préférence de 0,05 à 0,5%, tels que les esters propyliques, octyliques et dodécyliques de l'acide gallique, le butylhydroxytoluène et le butylhydroxyanisole ainsi que des parfums et des agents conservateurs tels que le parahydroxybenzoate de- méthyle ou de propyle. Ces additifs se trouvent selon leur solubilité, soit dans la phase grasse, soit dans la phase dispersée d'alcool polyhydrique.

La phase lipophile peut véhiculer un ou plusieurs actifs liposolubles communément utilisés dans les produits cosmétiques ou pharmaceutiques, à raison de 0,05 à 5% et de préférence de 0,5 à 3%.

Parmi ceux-ci, on peut citer les dérivés de vitamines tels que l'acétate de tocophérol et le palmitate de vitamine A, les acides gras essentiels, les sphingocérils, les céramides et les filtres solaires solubles.

Les compositions selon l'invention sont préparées, de façon générale, suivant un procédé comprenant les étapes suivantes :
- on chauffe la phase grasse constituée d'au moins une huile et d'au moins une cire, du composé organofluoré hydrocarboné et contenant éventuellement en dispersion des pigments et/ou des charges, à une température supérieure à la température la plus élevée de fusion des cires (température finissante), et
- on chauffe éventuellement, d'autre part, l'alcool polyhydrique contenant éventuellement des additifs solubles à la même température et on mélange les deux constituants.

L'émulsion ainsi obtenue est ensuite coulée dans un moule approprié.

L'invention concerne également l'utilisation des dispersions de l'invention dans les domaines de la cosmétique et de la dermatologie.

### EXEMPLES DE PREPARATION

### EXEMPLE A

### 1-(2'-F-hexyléthylthio) 3-(2''-éthylhexyloxy)-2-propanol

A la température de 25°C, sous agitation et sous courant d'azote, on ajoute à 152 g de 2-F-hexyléthanethiol, 3,6 g d'une solution méthanolique de méthylate de sodium (environ 30% - 5,54 meq g⁻¹) en une minute.

Le mélange est chauffé à 70°C. On évapore sous vide le méthanol présent dans le milieu.

Le 2-éthylhexylglycidyléther (74,4 g) est ensuite ajouté goutte-à-goutte en une heure. On maintient la température du mélange entre 60 et 70°C au cours de l'addition de l'époxyde.

A la fin de l'addition, la température est amenée à 25°C.

Le mélange est neutralisé à l'aide de 20 ml de HCl Normal.

Le 1-(2'-F-hexyléthylthio)3-(2''-éthylhexyloxy)2-propanol est séparé par distillation : Eb = 141°C/66,5 Pa.

On obtient 175 g (77%) d'une huile translucide incolore.

| Analyse élémentaire : | | | | |
|---|---|---|---|---|
| | % C | % H | % S | % F |
| Calculé | 40,28 | 4,80 | 5,66 | 43,60 |
| Mesuré | 40,37 | 4,82 | 5,55 | 43,74 |

### EXEMPLE B

### 1-(2'-F-octyléthylthio)2-hexanol

Selon le mode opératoire décrit à l'exemple A, on condense en 1 heure, 30 g (0,3 mole) de 1,2-epoxyhexane avec 144 g (0,3 mole) de 2-F-octyléthanethiol en présence de 2,7 g de solution méthanolique de méthylate de sodium (5,65 meq g⁻¹).

En fin de réaction, le mélange est neutralisé par 15 ml de HCl Normal.

Après distillation (154°C/133 Pa), on obtient 115 g d'un solide blanc amorphe qui est le 1-(2'-F-octyléthylthio)2-hexanol.
Rendement = 67%.
Point de fusion = 45°C.

| Analyse élémentaire : | | | | |
|---|---|---|---|---|
| | % C | % H | % S | % F |
| Calculé | 33,11 | 2,95 | 5,53 | 55,65 |
| Mesuré | 33,26 | 2,93 | 5,30 | 55,60 |

### EXEMPLES DE FORMULATION

### EXEMPLE 1 - Rouge à lèvres

| | |
|---|---|
| - Huile de ricin | 13,75 g |
| - Huile de sésame | 13,75 g |
| - Lanoline acétylée | 30 g |
| - Butylhydroxytoluène | 0,2 g |
| - Cire d'abeilles | 8 g |
| - Cire de Carnauba | 6 g |
| - Phosphate de trioléyle | 1 g |
| - Glycérine | 16 g |
| - Oxyde de titane | 2 g |
| - FD & C Yellow 6 Aluminium Lake | 3 g |
| - D & C Red 7 Calcium Lake | 5,8 g |
| - 1-(2'-F-hexyléthylthio)3-(2''-éthyl hexyloxy) 2-propanol (Exemple A) | 0,5 g |
| - Parfum qs | |

### EXEMPLE 2 - Rouge à lèvres

| | |
|---|---|
| - Huile de ricin | 13 g |
| - Huile de sésame | 13 g |
| - Lanoline acétylée | 30 g |
| - Butylhydroxytoluène | 0,2 g |
| - Cire d'abeilles | 8 g |
| - Cire de Carnauba | 6 g |
| - Phosphate de trioléyle | 1 g |
| - Glycérine | 16 g |
| - Oxyde de titane | 2 g |
| - FD & C Yellow 6 Aluminium Lake | 3 g |
| - D & C Red 7 Calcium Lake | 5,8 g |
| - 1-(2'-F-hexyléthylthio)3-(2''-éthyl hexyloxy) 2-propanol (Exemple A) | 2 g |
| - Parfum qs | |

### EXEMPLE 3 - Rouge à lèvres

| | |
|---|---|
| - Huile de ricin | 6 g |
| - Huile de sésame | 6 g |
| - Lanoline acétylée | 6 g |
| - Butylhydroxytoluène | 0,2 g |
| - Cire d'abeilles | 8 g |
| - Cire de Carnauba | 6 g |
| - Phosphate de trioléyle | 1 g |
| - Glycérine | 16 g |
| - Oxyde de titane | 2 g |
| - FD & C Yellow 6 Aluminium Lake | 3 g |
| - D & C Red 7 Calcium Lake | 5,8 g |
| - 1-(2'-F-hexyléthylthio)3-(2''-éthyl hexyloxy) 2-propanol (Exemple A) | 40 g |
| - Parfum qs | |

### EXEMPLE 4 - Rouge à lèvres

| | |
|---|---|
| - Triglycérides d'acides caprique/caprylique, vendus sous la dénomination de "MIGLYOL 812" par la Société HULS | 9 g |
| - Huile de sésame | 14 g |
| - Lanolate d'isopropyle | 34 g |
| - Butylhydroxytoluène | 0,2 g |
| - Cire de Carnauba | 5 g |
| - Cire de polyéthylène | 8 g |
| - Glycérine | 16 g |
| - Oxyde de titane | 2 g |
| - FD & C Yellow 6 Aluminium Lake | 3 g |
| - D & C Red 7 Calcium Lake | 5,8 g |
| - 1-(2'-hexyléthylthio)3-(2''-éthyl hexyloxy) 2-propanol (Exemple A) | 3 g |
| - Parfum qs | |

### EXEMPLE 5 - Rouge à lèvres

| | |
|---|---|
| - Huile de sésame | 15 g |
| - Huile de vaseline | 23 g |
| - Lanoline | 20 g |
| - Butylhydroxytoluène | 0,2 g |
| - Cire microcristalline | 10 g |
| - Cire de Carnauba | 3 g |
| - Lécithine de soja | 1 g |
| - Glycérine | 12 g |
| - D & C Red 27 | 5 g |
| - Oxyde de fer noir | 0,2 g |
| - D & C Red 7 Calcium Lake | 5,6 g |
| - Oléate de 1,1,2,2-tétrahydroheptadécafluoro-1-décanol (Nofable FO-9982 de Nippon Oil fats) | 5 g |

### EXEMPLE 6 - Rouge à lèvres

| | |
|---|---|
| - Huile de jojoba | 7 g |
| - Huile de sésame | 10 g |
| - Lanolate d'isopropyle | 16 g |
| - Butylhydroxytoluène | 0,2 g |
| - Ozokérite | 8 g |
| - Cire d'abeilles | 8 g |
| - Polybutène (INDOPOL de la Société AMOCO) | 10 g |
| - Phosphate de trioléyle | 2 g |
| - Polyglycérine 500 | 16 g |
| - FD & C Yellow 6 Aluminium Lake | 1 g |
| - D & C Red 27 | 9 g |
| - Dioxyde de titane | 2,8 g |
| - 1-(2'-F-hexyléthylthio)3-(2''-hexyléthyloxy) 2-propanol (Exemple A) | 10 g |

### EXEMPLE 7 - Rouge à lèvres

| | |
|---|---|
| - Huile de ricin | 5 g |
| - Huile de vaseline | 20 g |
| - Lanoline | 10 g |
| - Lanolate d'isopropyle | 25 g |
| - Lanolate de magnésium | 4 g |
| - Butylhydroxytoluène | 0,2 g |
| - Cire de Carnauba | 2,8 g |
| - Cire microcristalline | 15 g |
| - Glycérine | 3 g |
| - Perfluorohexyl-2 éthanol | 5 g |
| - Pigments | 10 g |

### EXEMPLE 8 - Rouge à lèvres

| | |
|---|---|
| - Huile de jojoba | 7 g |
| - Huile de sésame | 10 g |
| - Lanolate d'isopropyle | 9 g |
| - Butylhydroxytoluène | 0,2 g |
| - Ozokérite | 8 g |
| - Cire d'abeilles | 8 g |
| - Polybutène | 10 g |
| - Phosphate de trioléyle | 2 g |
| - Polyglycérine 500 | 25 g |
| - FD & C Yellow 6 Aluminium Lake | 1 g |
| - D & C Red 27 | 9 g |
| - Dioxyde de titane | 2,8 g |
| - 1-(2'-F-hexyléthylthio)3-(2''-hexyléthyloxy) 2-propanol (Exemple A) | 8 g |

### EXEMPLE 9 - Fond de teint

| | |
|---|---|
| - Cire microcristalline | 8 g |
| - Cire de Carnauba | 4 g |
| - Octyl palmitate | 14,5 g |
| - Isoparaffine | 23 g |
| - Lanolate d'isopropyle | 4 g |
| - Parahydroxybenzoate de propyle | 0,2 g |
| - Oxyde de fer jaune | 1 g |
| - Oxyde de fer brun | 0,37 g |
| - Oxyde de fer noir | 0,15 g |
| - Oxyde de titane | 5,47 g |
| - Oxyde de zinc | 3 g |
| - Talc | 3 g |
| - Poudre de nylon | 3 g |
| - Microsphères vendues sous la dénomination de "EXPANCEL DE" par la Société KEMANORD PLAST AB | 1,2 g |
| - Diméthicone | 0,3 g |
| - Polylaurate de vinyle | 12,5 g |
| - Glycérine | 12 g |
| - Lanolate de magnésium | 1 g |
| - 1-(2'-F-octyléthylthio)2-hexanol (Exemple B) | 3 g |
| - Parfum | 0,31 g |

### EXEMPLES DE COMPARAISON

### EXEMPLE COMPARATIF 1

| | |
|---|---|
| - Huile de ricin | 14 g |
| - Huile de sésame | 14 g |
| - Lanoline acétylée | 30 g |
| - Butylhydroxytoluène | 0,2 g |
| - Cire d'abeilles | 8 g |
| - Cire de Carnauba | 6 g |
| - Phosphate de trioléyle (par exemple l'Hosphat K0 300 de Hoechst) | 1 g |
| - Glycérine | 16 g |
| - Oxyde de titane | 2 g |
| - FD & C Yellow 6 Aluminium Lake | 3 g |
| - D & C Red 7 Calcium Lake | 5,8 g |
| - Parfum qs | |

### EXEMPLE COMPARATIF 2

| | |
|---|---|
| - Triglycérides d'acides caprique/caprylique, vendus sous la dénomination de "MIGLYOL 812" par la Société HULS | 10 g |
| - Huile de sésame | 15 g |
| - Lanoline d'isopropyle | 35 g |
| - Butylhydroxytoluène | 0,2 g |
| - Cire de Carnauba | 5 g |
| - Cire de polyéthylène | 8 g |
| - Glycérine | 16 g |
| - Oxyde de titane | 2 g |
| - FD & C Yellow 6 Aluminium Lake | 3 g |
| - D & C Red 7 Calcium Lake | 5,8 g |
| - Parfum qs | |

Au bout d'une journée, les sticks des exemples comparatifs exsudent en se couvrant de gouttelettes.

Après 1 mois, les sticks, selon l'invention, ne présentent toujours pas de gouttelettes.

## Revendications

1. Dispersion solide anhydre à base d'un corps gras et d'un alcool polyhydrique, caractérisée en ce qu'elle comporte, par rapport au poids total de la dispersion :
- 20 à 95% en poids et de préférence 40 à 85% en poids du corps gras constitué de 10 à 50% en poids d'au moins une cire de point de fusion supérieur à 55°C,
- 0,5 à 50% en poids d'un alcool polyhydrique dispersé, de préférence 4 à 40% en poids, et
- 0,1 à 50% en poids et de préférence 0,5 à 20% en poids d'au moins un composé organofluoré hydrocarboné.

2. Dispersion selon la revendication 1, caractérisée en ce que les fluorohydrocarbures ont la formule (I) :
(R_{F})ₓ - (A)_{y} - (R_{H})_{z} (I)
dans laquelle :
x représente 1, 2 ou 3,
y représente 0 ou 1,
z représente 0, 1, 2 ou 3,
à la condition que y et z ne soient pas simultanément 0, et que lorsque z est 0, x est 2 ou 3,
R_{F} représente un radical fluoré aliphatique ou aromatique, saturé ou insaturé, à chaîne linéaire, ramifiée ou cyclique, cette chaîne pouvant être fonctionnalisée et/ou être interrompue par des atomes divalents tels que l'oxygène ou le soufre, ou trivalents tels que l'azote et/ou substituée par des atomes d'hydrogène ou d'autres atomes d'halogène, à la condition que, pour deux atomes de carbone du squelette, ne soit pas présent plus d'un de ces substituants autres que le fluor,
R_{H} représente un radical hydrocarboné aliphatique ou aromatique, saturé ou insaturé, à chaîne linéaire, ramifiée ou cyclique, cette chaîne pouvant être fonctionnalisée et/ou interrompue par un ou plusieurs atomes divalents tels que l'oxygène ou le soufre ou par un ou plusieurs atomes trivalents comme l'azote,
A représente un radical di, tri ou quadrivalent tel que 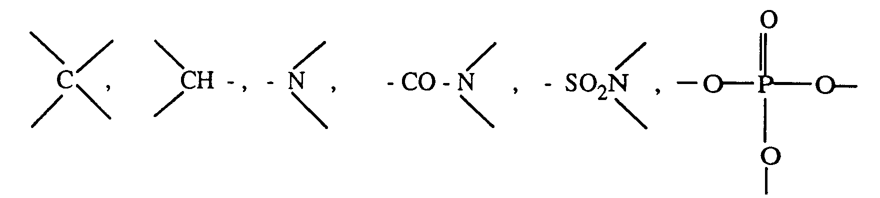 les structures cycliques, aliphatiques ou aromatiques ou les insaturations éthyléniques.

3. Dispersion selon l'une des revendications 1 ou 2, caractérisée en ce que les fluorocarbures ont pour formule la formule (III) suivante :
R₁ - (CH₂)ₙ - X - [C₃H₅(OH)] - (Y)ₓ - R₂ (III)
dans laquelle C₃H₅(OH) représente les structures : 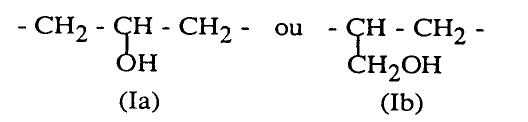
R₁ représente un radical alkyle perfluoré en C₄-C₂₀ ou un mélange de radicaux alkyle perfluorés en C₄-C₂₀;
R₂ représente un radical alkyle linéaire ou ramifié en C₁-C₂₂ ou un mélange de radicaux alkyle linéaires ou ramifiés en C₁-C₂₂ ou un radical aryle ou aralkyle;
n est compris entre 0 et 4;
X représente O, S, 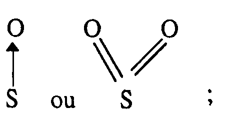
x représente O ou 1 ;
Y représente O, S, 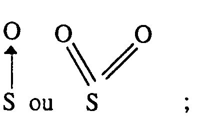
sous réserve que lorsque X = 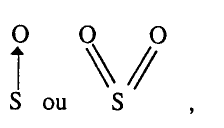 Y n'est pas 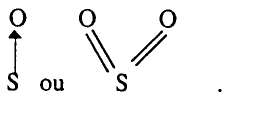
ou la formule (VI) : 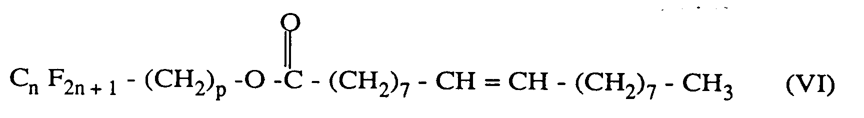 dans laquelle n est un nombre entier égal à 6 ou 8 et p est 1 ou 2.

4. Dispersion selon la revendication 1 ou 3, caractérisée en ce que les fluorocarbures ont un taux de substitution compris entre 10 et 90%, et de préférence 20 et 80%.

5. Dispersion selon l'une des revendications 1 à 4, caractérisée en ce que l'alcool polyhydrique est un composé ayant de 2 à 8 atomes de carbone et de 2 à 6 fonctions hydroxyle, de préférence l'éthylèneglycol, le glycérol, le propane-diol-1,2, la diglycérine, l'érythritol, l'arabitol, l'adonitol, le sorbitol ou le dulcitol ou un polyéther alcool de poids moléculaire moyen compris entre 150 et 600.

6. Dispersion selon la revendication 5, caractérisée par le fait que lorsque le composé fluorohydrocarboné est fonctionnalisé avec des groupements OH, SH, NH ou NH₂, l'alcool polyhydrique comporte au moins trois fonctions hydroxyle.

7. Dispersion selon l'une des revendications 1 à 6, caractérisée par le fait qu'elle contient jusqu'à 30% et de préférence de 0,1 à 20% en poids total par rapport au poids total de la dispersion d'un agent tensio-actif.

8. Dispersion selon l'une des revendications 1 à 7, caractérisée par le fait que l'alcool polyhydrique contient des actifs hydrosolubles à une concentration comprise entre 0,05 et 5% en poids par rapport au poids total de la dispersion.

9. Dispersion selon l'une des revendications 1 à 8, caractérisée par le fait qu'elle contient au moins un polymère susceptible de la stabiliser en atmosphère humide.

10. Dispersion selon l'une des revendications 1 à 9, caractérisée par le fait qu'elle contient jusqu'à 30%, de préférence de 0,1 à 20% en poids par rapport au poids total de la dispersion d'au moins un pigment.

11. Dispersion selon l'une des revendications 1 à 10, caractérisée par le fait qu'elle contient jusqu'à 10% en poids par rapport au poids total de la dispersion d'au moins une charge minérale.

12. Utilisation d'une dispersion selon l'une des revendications 1 à 11 pour la préparation de compositions cosmétiques.

## Claims

1. Anhydrous solid dispersion based on a fatty substance and a polyhydric alcohol, characterised in that it comprises, by weight with respect to the total dispersion weight:
- 20 to 95%, preferably 40 to 85% of a fatty substance constituted by 10 to 15% by weight of at least one wax having a melting point greater than 55°C,
- 0.5 to 50%, preferably 4 to 40% of a dispersed polyhydric alcohol, and
- 0.1 to 50%, preferably 0.5 to 20% of at least one organofluorinated hydrocarbon compound.

2. Dispersion according to claim 1 characterised in that the fluorohydrocarbons have the following formula (I):
(R_{F})ₓ - (A)_{y} - (R_{H})_{z} (I)
wherein
x is 1, 2 or 3,
y is 0 or 1,
z is 0, 1, 2 or 3,
provided that y and z are not simultaneously 0 and that when z is 0 x is 2 or 3,
R_{F} is an aliphatic or aromatic, saturated or unsaturated fluorinated radical with a linear, branched or cyclic chain capable of being functionalised and/or interrupted by divalent atoms such as oxygen or sulphur or trivalent atoms such as nitrogen and/or substituted by hydrogen atoms and other halogen atoms, provided that, for two carbon atoms of the backbone, no more than one of these substituents other than fluorine is present,
R_{H} is an aliphatic or aromatic, saturated or unsaturated hydrocarbon radical with a linear, branched or cyclic chain capable of being functionalised and/or interrupted by one or more divalent atoms such as oxygen or sulphur or by one or more trivalent atoms such as nitrogen,
A is a di-, tri- or quadrivalent radical such as 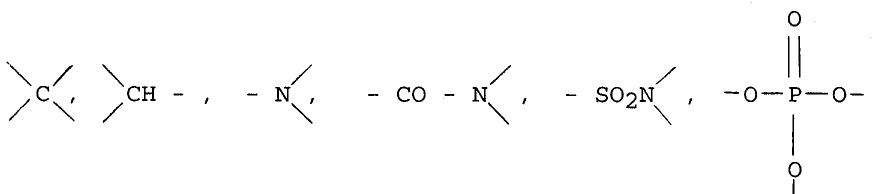 cyclic, aliphatic or aromatic structures or unsaturated ethylene structures.

3. Dispersion according to claim 1 or claim 2 characterised in that the fluorohydrocarbons have the following formula (III):
R₁ - (CH₂)ₙ - X - [C₃H₅(OH)] - (Y)ₓ - R₂ (III)
wherein C₃H₅(OH) is the structure: 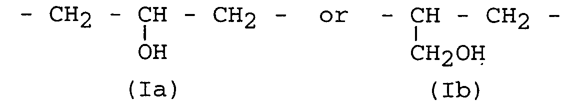
R₁ is a C₄-C₂₀ perfluorinated radical or a mixture of C₄-C₂₀ perfluorinated alkyl radicals;
R₂ is a linear or branched C₁-C₂₂ alkyl radical or a mixture of linear or branched C₁-C₂₂ alkyl radicals or an aryl or aralkyl radical;
n is between 0 and 4;
X is O, S, 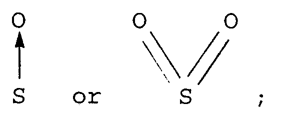
x is 0 or 1;
Y is O, S, 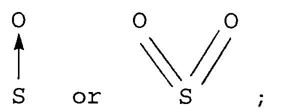
provided that when X is 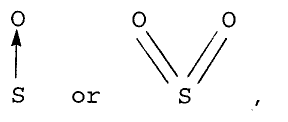
Y is not 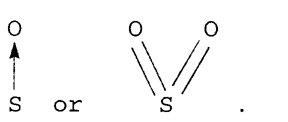
or formula (VI): 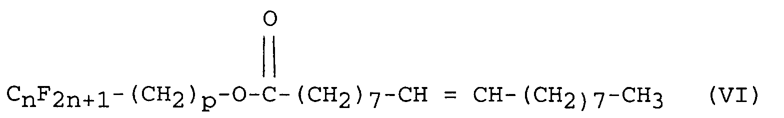 wherein n is 6 or 8 and p is 1 or 2.

4. Dispersion according to claim 1 or claim 3 characterised in that the fluorocarbons have a substitution ratio of between 10 and 90%, preferably between 20 and 80%.

5. Dispersion according to any one of claims 1 to 4 characterised in that the polyhydric alcohol is a compound having two to eight carbon atoms and two to six hydroxyl functions, preferably ethylene glycol, glycerol, propane-1,2-diol, diglycerine, erythyritol, arabitol, adonitol, sorbitol or dulcitol or a polyether alcohol having an average molecular weight of between 150 and 600.

6. Dispersion according to claim 5 characterised in that when the fluorohydrocarbon compound is functionalised with OH, SH, NH or NH₂ groups the polyhydric alcohol comprises at least three hydroxyl functional groups.

7. Dispersion according to any one of claims 1 to 6 characterised in that it contains up to 30% and preferably from 0.1 to 20% by weight with respect to the total dispersion weight of a surfactant.

8. Dispersion according to any one of claims 1 to 7 characterised in that the polyhydric alcohol contains hydrosoluble active ingredients in a concentration between 0.05 and 5% by weight with respect to the total dispersion weight.

9. Dispersion according to any one of claims 1 to 8 characterised in that it contains at least one polymer for stabilising it in a damp atmosphere.

10. Dispersion according to any one of claims 1 to 9 characterised in that it contains up to 30%, preferably 0.1 to 20% by weight with respect to the total dispersion weight of at least one pigment.

11. Dispersion according to any one of claims 1 to 10 characterised in that it contains up to 10% by weight with respect to the total dispersion weight of at least one mineral filler.

12. Use in the preparation of cosmetic compositions of a dispersion as claimed in any one of claims 1 to 11.

## Patentansprüche

1. Feste wasserfreie Dispersion auf Basis eines Fettkörpers und eines polyhydrischen Alkohols,
dadurch **gekennzeichnet**, daß
sie, bezogen auf das Gesamtgewicht der Dispersion, enthält:
- 20 bis 95 und vorzugsweise 40 bis 85 Gew.% des Fettkörpers aus 10 bis 50 Gew.% mindestens eines Wachses eines Schmelzpunktes von oberhalb 55°C,
- 0,5 bis 50 und vorzugsweise 4 bis 40 Gew.% eines dispergierten polyhydrischen Alkohols, und
- 0,1 bis 50 und vorzugsweise 0,5 bis 20 Gew.% mindestens einer Organofluor-Kohlenwasserstoffverbindung.

2. Dispersion gemäß Anspruch 1,
dadurch **gekennzeichnet**, daß
die Fluorkohlenwasserstoffe die Formel (I) aufweisen:
(R_{F})ₓ - (A)_{y} - (R_{H})_{z} (I)
worin gilt:
x stellt 1, 2 oder 3 dar,
y stellt 0 oder 1 dar,
z stellt 0, 1, 2 oder 3 dar,
mit der Maßgabe, daß y und z nicht gleichzeitig 0 sind, und daß, wenn z 0 ist, x 2 oder 3 ist,
R_{F} stellt einen aliphatischen oder aromatischen, gesättigten oder ungesättigten fluorhaltigen Rest mit linearer, verzweigter oder zyklischer Kette dar, wobei die Kette durch zweiwertige Atome wie Sauerstoff oder Schwefel oder durch dreiwertige Atome wie Stickstoff funktionalisiert und/oder unterbrochen und/oder mit Wasserstoffatomen oder anderen Halogenatomen substituiert sein kann, mit der Maßgabe, daß, für zwei Kohlenstoffatome des Gerüsts, nicht mehr als einer derjenigen Substituenten vorhanden ist, die sich von Fluor unterscheiden,
R_{H} stellt einen aliphatischen oder aromatischen, gesättigten oder ungesättigten Kohlenwasserstoffrest mit linearer, verzweigter oder zyklischer Kette dar, wobei diese Kette durch ein oder mehrere zweiwertige Atome wie Sauerstoff oder Schwefel oder durch ein oder mehrere dreiwertige Atome wie Stickstoff funktionalisiert und/oder unterbrochen sein kann, A stellt einen zwei-, drei- oder vierwertigen Rest wie: 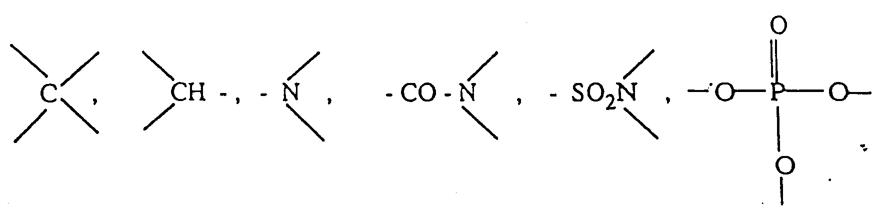 aliphatische oder aromatische zyklische Strukturen oder ethylenisch ungesättigte Strukturen dar.

3. Dispersion gemäß einem der Ansprüche 1 oder 2,
dadurch **gekennzeichnet**, daß
die Fluorkohlenwasserstoffe die folgende Formel (III):
R₁ - (CH₂)ₙ - X - [C₃H₅(OH)] - (Y)ₓ - R₂ (III)
worin C₃H₅(OH) die Strukturen darstellt: 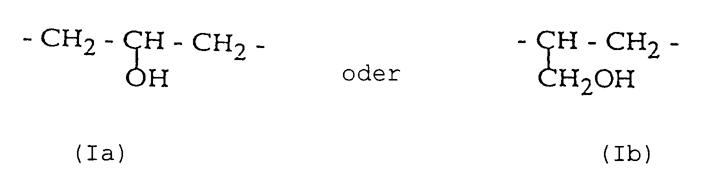 und worin ferner gilt:
R₁ stellt einen perfluorierten C₄₋₂₀-Alkylrest oder eine Mischung aus perfluorierten C₄₋₂₀-Alkylresten dar;
R₂ stellt einen linearen oder verzweigten C₁₋₂₂-Alkylrest oder eine Mischung aus linearen oder verzweigten C₁₋₂₂-Alkylresten oder einen Aryl- oder Aralkylrest dar;
n beträgt 0 bis 4;
X stellt dar: O, S, 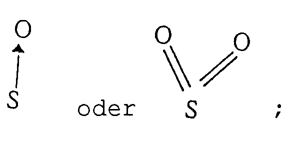
x stellt 0 oder 1 dar;
Y stellt dar: O, S, 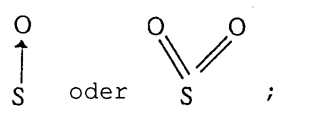
mit der Maßgabe, daß, wenn X = 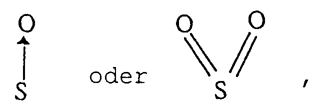
Y nicht 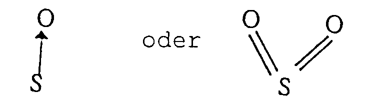 ist,
oder die Formel (VI) aufweisen: 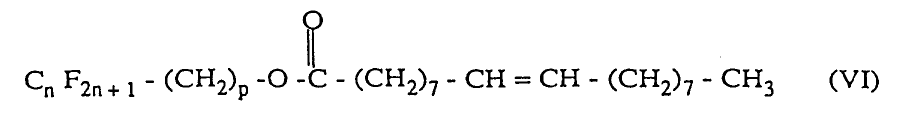 worin n eine ganze Zahl von 6 oder 8 und p 1 oder 2 sind.

4. Dispersion gemäß Anspruch 1 oder 3,
dadurch **gekennzeichnet**, daß
die Fluorkohlenwasserstoffe einen Substitutionsgrad von 10 bis 90 und vorzugsweise von 20 bis 80% aufweisen.

5. Dispersion gemäß einem der Ansprüche 1 bis 4,
dadurch **gekennzeichnet**, daß
der polyhydrische Alkohol eine Verbindung mit 2 bis 8 Kohlenstoffatomen und 2 bis 6 Hydroxylfunktionen und vorzugsweise Ethylenglycol, Glycerin, Propandiol-1,2, Diglycerin, Erythrit, Arabit, Adonit, Sorbit oder Dulcit oder ein Polyetheralkohol mit einem mittleren Molekulargewicht von 150 bis 600 ist.

6. Dispersion gemäß Anspruch 5,
dadurch **gekennzeichnet**, daß,
wenn die Fluorkohlenwasserstoffverbindung mit OH-, SH-, NH- oder NH₂-Gruppen funktionalisiert ist, der polyhydrische Alkohol mindestens 3 Hydroxylfunktionen aufweist.

7. Dispersion gemäß einem der Ansprüche 1 bis 6,
dadurch **gekennzeichnet**, daß
sie bis zu 30 und vorzugsweise 0,1 bis 20 Gew.% , bezogen auf das Gesamtgewicht der Dispersion, eines oberflächenaktiven Mittels enthält.

8. Dispersion gemäß einem der Ansprüche 1 bis 7,
dadurch **gekennzeichnet**, daß
der polyhydrische Alkohol wasserlösliche Wirkstoffe in einer Konzentration von 0,05 bis 5 Gew.% enthält, bezogen auf das Gesamtgewicht der Dispersion.

9. Dispersion gemäß einem der Ansprüche 1 bis 8,
dadurch **gekennzeichnet**, daß
sie mindestens ein Polymer enthält, das sich dazu eignet, die Dispersion an feuchter Atmosphäre zu stabilisieren.

10. Dispersion gemäß einem der Ansprüche 1 bis 9,
dadurch **gekennzeichnet**, daß
sie bis zu 30 und vorzugsweise 0,1 bis 20 Gew.%, bezogen auf das Gesamtgewicht der Dispersion, mindestens eines Pigments enthält.

11. Dispersion gemäß einen der Ansprüche 1 bis 10,
dadurch **gekennzeichnet**, daß
sie bis zu 10 Gew.%, bezogen auf das Gesamtgewicht der Dispersion, mindestens eines mineralischen Beaufschlagungsmittels enthält.

12. Verwendung einer Dispersion gemäß einem der Ansprüche 1 bis 11 zur Herstellung kosmetischer Zusammensetzungen.
